# EUROPEAN PATENT SPECIFICATION

(11) **EP 0 631 498 B1**
(45) Date of publication and mention of the grant of the patent: **11.06.1997**
(21) Application number: 93907822.6
(22) Date of filing: 19.03.1993
(51) Int. Cl.: A61F 5/448, A61F 5/44

(54) **A CONVEX RING**
KONVEXER RING
ANNEAU CONVEXE

(30) Priority: 20.03.1992 DK 371/92
(43) Date of publication of application: 04.01.1995
(73) Proprietor: COLOPLAST A/S, DK-3060 Espergaerde (DK)
(72) Inventor: OLSEN, Hans, DK-2700 Broenshoej (DK)
(74) Representative: Christiansen, Ejvind
(86) International application number: DK9300101
(87) International publication number: WO9318725

(56) References cited:
- EP-A- 0 317 326
- EP-A- 0 479 573
- WO-A-91/01118

## Description

The present invention concerns a convex ring for use in connection with an ostomy appliance, consisting of at least two parts adapted to be coupled together, a first coupling part being intended for attachment around the patient's stoma.

Below an ostomy patient or an ostomist denotes a person having a colostomy, an ileostomy or a urostomy. In such persons the colon, the ileum or the ureter has been exposed surgically such that the waste products of the body, which are conveyed through these organs, are discharged through an artificial opening and are collected in a collection bag, which is ordinarily adhered to the skin by means of an adhesive plate with an opening surrounding the stoma.

It is frequently seen in ostomy patients that the stoma or its closest surroundings are recessed or are positioned in a crater or a cavity with respect to the rest of the skin surface that surrounds the stoma. For such patients it is expedient to use an ostomy appliance where the adhesive surface around the opening of the appliance for receiving the stoma has a part which protrudes toward the user with a view to enabling the adhesive face of the ostomy appliance to engage and adhere to the skin everywhere in the crater or the cavity. In particular, it is important that the ostomy appliance adheres to the skin as closely to the stoma as possible, and this location is most frequently the one lying deepest. The shape of the forwardly protruding part of the adhesive face may e.g. be domed or conical.

Throughout the specification the term convex has this broad meaning irrespective of the actual embodiment.

Ostomy equipment having a convex part protruding toward the user is known from e.g. EP patent applications 415282, 415592, 317326 and 416397.

The two first-mentioned patent applications concern ostomy equipment having an integrated protruding convex part. The ostomy equipment consists of a bag part and an attachment part, said parts being either interconnectible or being integrated. The attachment part, which is intended for attachment by adhesion around the patient's stoma, has a central opening and is coated with a layer of an adhesive material on the proximal side (the side facing the user), which has a radially inner and outer section, the radially inner section being relatively rigid and having a convex shape.

Ostomy equipment of the above-mentioned type can only be used by ostomists having a recessed stoma. For ostomists without a recessed stoma it is thus necessary to manufacture another ostomy equipment or at least another attachment part without a convex protruding part, which necessarily adds to the costs of the product.

The ostomy equipment described in EP patent applications 317326 and 416397 likewise consists of a bag part and an attachment part, said parts being either interconnectible or being integrated, as well as a convex adapter. The adapter, which is intended to be positioned around the patient's stoma, is relatively rigid and has a central opening and is coated with a layer of an adhesive material on its promixal side (the side facing the user), which has a radially inner and outer section, the radially inner section around the central opening having a convex shape.

The attachment part, which is likewise coated with an adhesive layer, can be attached to the distal side of the adapter or can be attached directly to the skin around the stoma, if the ostomy equipment is to be used without an adapter. Thus, this type of ostomy equipment can be used with or without a convex adapter, according to whether the ostomist has a recessed or a non-recessed stoma.

However, ostomy equipment having an adapter of the last-mentioned type is very expensive because of the use of an excessive amount of adhesive material which is to be applied in several adhesive layers, and this application may be difficult in terms of handling.

The production of all of the above-mentioned ostomy appliances is moreover difficult, because it may be rather problematic to apply an adhesive layer on a convex surface without air pockets being formed. It is moreover very difficult to apply a removable cover layer on the surface of the adhesive layer without creating chinks or fissures in the paper. The last-mentioned problem has been solved in EP patent application 415282 by replacing the paper cover layer by a removable plastics cover layer that can be given the convex shape by vacuum moulding. However, this solution is very expensive.

The applicant's own patent application WO 91/01118 describes a convex ring for use in connection with an ostomy appliance consisting of at least two parts adapted to be coupled together, wherein a first coupling part is intended for attachment around the stoma and a second coupling part carries a collection bag. The convex ring, which is of a relatively rigid material, has a radial extent and a central opening as well as a proximal side and a distal side, said proximal side having a convex surface. In its radially outer annular edge the ring is shaped so as to engage only a flange or a plurality of knobs on a radially inner side of the first coupling part of the ostomy appliance, the convex ring being thus retained between the knobs or the flange and a film which is coated with an adhesive layer, by means of which the ostomy appliance can be attached to the ostomist's skin surface.

Thus, the convex ring is not coated or in contact with an adhesive layer, which does not only reduce the costs of the product considerably, but also makes it possible to use the convex ring again, and additionally the ostomy appliance can be used with or without a convex ring as desired by the ostomist.

In use the convex ring is mounted in the first coupling part either before or after the coupling part has been attached around the stoma. The convex ring is thus pressed down into the first coupling part from the distal side of said part so that the adhesive layer of the coupling part in the area located or intended to be located closest around the stoma, is given a convex shape.

Since the adhesive layer is thus deformed by the mounting of the convex ring, it may be very difficult to mount the ring when the removable cover layer is applied to the adhesive layer, because this cover layer offers considerably greater resistance to deformation than the adhesive layer. It is therefore expedient to attach the first coupling part around the stoma before the convex ring is mounted.

The ostomist thus first adheres the first coupling part to the skin surface around the crater or the cavity. Then the convex ring is pressed down into the central portion of the first coupling part, so that the radially outer annular edge of the convex ring engages a flange or a plurality of knobs on the radially inner side of the coupling part, thereby imparting to the central portion of the adhesive face of the coupling part a convex shape which protrudes down into the cavity or the crater and adheres to the skin surface closest around the stoma.

However, the mounting of the convex ring involves some difficulties, because it must necessarily be pressed down into the coupling part by a certain force to engage the flange or the knobs on the central inner side of the coupling part. Thus, the ostomist can inadvertently press the convex ring too far down into the stoma, which may be very painful, and the ostomist is liable to press the outer annular edge of the convex ring between the adhesive layer and the rest of the coupling part so that the convex ring is positioned askew, which at worst can cause wounds on the stoma. Additionally, the radial extent between the inner opening and the annular edge of the outer periphery is very narrow, and it may be difficult to have sufficient space for a finger, and the finger, when pressing in the convex ring, is very liable to "slide" off the ring into the opening of the ring and to prick hard into the stoma, which may likewise cause pain and possibly wounds on the stoma.

Since the area in and around the stoma is always very crowded with bacteria, it is very difficult for such a wound to heal, and the risk of the wound going septic is very great. Further, when applying the convex ring it may be difficult for the ostomist to judge when the ring has been pressed sufficiently far down.

The object of the present invention is thus to provide a convex ring for use in connection with an ostomy appliance consisting of at least two parts adapted to be coupled together, a first coupling part being intended for attachment around the patient's stoma, said convex ring being relatively easy and safe to position in the first coupling part after said part has been attached around the stoma, while making it easy for the ostomist to judge whether the ring is positioned correctly.

This object is obtained by a convex ring in combination with an ostomy appliance which is of the types defined in the introductory portion of claim 1 and which is characterized by the features defined in the characterizing portion of claim 1.

The convex ring of the invention moreover has the advantage that it is relatively inexpensive in use, one reason being that it is inexpensive to produce, another that it can be reused repeatedly.

Distal and proximal sides, respectively, mean the side facing away from the user and the side facing toward the user, and distal and proximal directions, respectively, mean in a direction away from the user and in a direction toward the user. Inner and outer are radial directional statements, and inner side and outer side mean the inner side and outer side, respectively, of the cylindrical parts protruding in a distal or proximal direction.

The convex ring of the invention is thus in combination with an ostomy appliance consisting of at least two parts adapted to be coupled together, a first coupling part being intended for attachment around a stoma and a second coupling part that can carry a bag. The coupling parts may be adapted to be coupled together in a generally known manner, e.g. by mechanically engaging each other, by adhesion or by means of a third coupling ring, as known from the applicant's own patent applications WO 91/01118 and WO 91/01119. Other suitable coupling methods are e.g. those mentioned in EP patent application 463359 and US patent specification 4 460 363.

When constructing a convex ring according to the invention the ostomy appliance with which it is to be combined, should be taken into consideration, because the part protruding in the distal direction must be shaped so that it does not prevent the coupling parts of the ostomy appliance from being coupled together. Furthermore, the convex ring and the ostomy appliance, for which it is to be used, should preferably be constructed such that the coupling parts of the ostomy appliance can be coupled together independently of the presence of the convex ring, because in that case ostomists with and without a recessed stoma can use the same ostomy appliance.

The convex ring of the invention preferably consists of a semi-rigid or rigid polymer material. Suitable materials in particular include polypropylene, HD polyethylene and polystyrene.

The invention will be described more fully below with reference to the drawing, in which
fig. 1 shows the profile of a preferred embodiment of the convex ring of the invention, as well as a first coupling part of an ostomy appliance with which the convex ring may be coupled,
fig. 2 shows the convex ring of fig. 1 mounted in the first coupling part,
fig. 3 shows a section of a second preferred embodiment of the convex ring of the invention seen in perspective and mounted in an ostomy appliance,
fig. 4 shows the profile of a third embodiment of the convex ring of the invention as well as two coupling parts of an ostomy applicance with which the convex ring is combined,
fig. 5 shows the convex ring of fig. 4 mounted in the ostomy appliance, and
fig. 6 shows the profile of a fourth embodiment of the convex ring of the invention, mounted in a first coupling part of an ostomy appliance, as well as the second coupling part of the ostomy appliance.

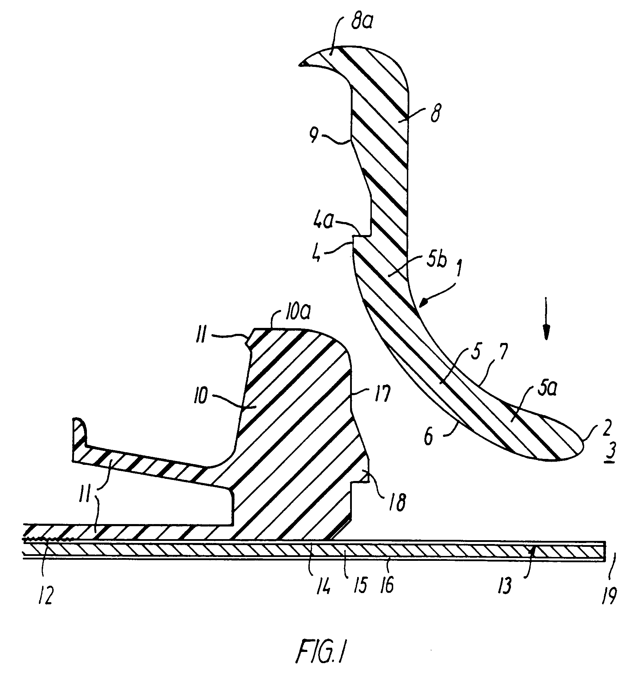

The convex ring 1 shown in figs. 1-2 has an inner periphery 2 surrounding an inner opening 3 having a circumference which substantially corresponds to or is slightly greater than the circumference of the stoma for which the convex ring 1 is to be used. The convex ring 1 moreover has an outer periphery 4 with an edge 4a. In the section 5 between the inner and outer peripheries 2, 4 the convex ring 1 has a proximal side 6 and a distal side 7. The ring 1 has a convex surface on its proximal side 6. The section 5 consists of inner and outer sections 5a, 5b. On the outer section 5b the convex ring 1 has a distally protruding part 8, which protrudes radially outwardly at its upper end 8a. The protruding part 8 has an outer side 9 with a profile fitting in a first coupling part 10. The first coupling part 10 has a distal surface 10a and coupling means 11 by which it can be coupled to the other coupling parts of the ostomy appliance. The first coupling part 10 is moreover secured by welding or glueing 12 to a film 14 of an adhesive plate 13 consisting of the film layer 14 and an adhesive layer 15 and having an inner opening 19. In fig. 1 the adhesive layer 15 is additionally coated with a removable cover layer 16. The coupling part has an inner side surface 17 corresponding to the outer side 9 of the protruding part. Thus, on its inner side 17 the coupling part has a protruding flange 18 capable of engaging the convex ring edge 4a, thereby retaining the convex ring 1 between the flange 18 and the adhesive plate 13.

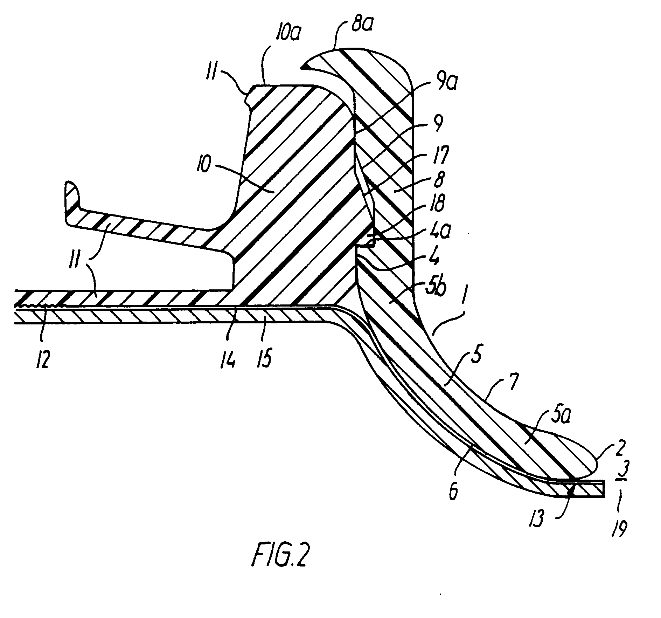

In use the ostomist first removes the cover layer 16 from the adhesive plate 13 of the coupling part 10, and then the adhesive plate is adhered to the skin surface surrounding the crater or the cavity around the stoma. The convex ring 1 is then pressed by applying a pressure to the upper end 8a of the protruding part until the upper end 8a of the protruding part abuts the coupling part 10. The upper end 8a of the protruding part may also be shaped so that it does not protrude radially. In that case the height of the protruding part 8 must be such that the upper end 8a is flush with the distal surface 10 of the coupling part when the convex ring is positioned correctly. Thus, the fingers, which are pressed against the upper end 8a in the mounting of the convex ring, provide a natural stop when they hit the surface 10a. The ring cannot be pressed further down and is now positioned correctly. It will be seen in fig. 2 how the convex ring and the coupling part engage each other, and it will be seen that the adhesive plate in its inner section, i.e. the section closest to the opening 19, has obtained a convex surface, so that the adhesive layer 15 adheres to the skin surface in the cavity or the crater surrounding the stoma. It moreover appears from fig. 2 that the outer side 9 of the protruding part of the convex ring is not in contact with the inner side 17 of the coupling part in its entire extent, but just at the edge 4a and an upper area 9a. This additionally reduces the friction in the mounting of the convex ring.

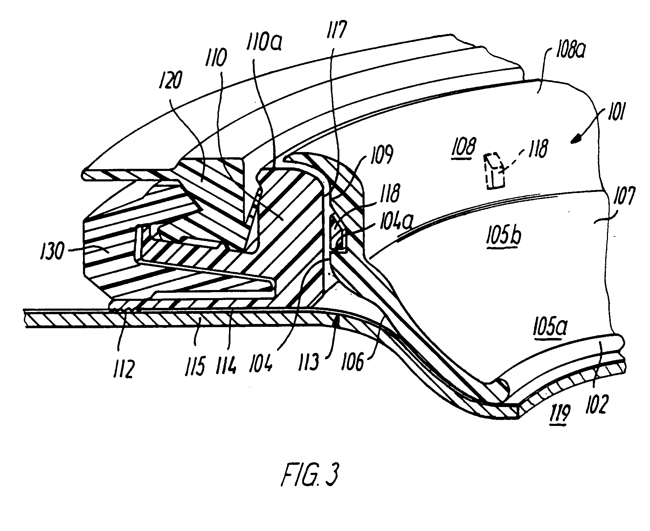

Fig. 3 shows a sketch of a second preferred embodiment of the convex ring of the invention mounted in an ostomy appliance. The ostomy appliance consists of three coupling parts. A first coupling part 110 having a distal surface 110a and an inner side 117 with a plurality of protruding knobs 118. The coupling part is secured by welding or glueing 112 to a film 114 of an adhesive plate 113 consisting of the film 114 and an adhesive layer 115 and having an inner opening 119, a second coupling part 120 carrying in a known manner a collection bag (not shown), and a third coupling part 130 which is a locking ring and serves to keep the two first-mentioned coupling parts together. This ostomy appliance and the coupling between the individual parts are described more fully in the applicant's patent applications WO 91/01118 and WO 91/01119. The convex ring 101 has substantially the same structure as the convex ring 1 shown in figs. 1-2, but the profile is slightly different. Thus, the convex ring 101 has an inner periphery 102 and an outer periphery 104 with an edge 104a and inner and outer sections 105a, 105b between the inner and outer peripheries, a proximal side 106 and a distal side 107, said inner section 105a having a convex shape on its proximal side 106. On the outer section 105b the convex ring has a protruding part 108 which protrudes radially outwardly at its upper end 108a. The protruding part 108 has an outer side 109 fitting in the coupling part 110 and corresponding to the inner side 117 thereof in the same manner as is described in the discussion of figs. 1-2.

The coupling part 110 and the convex ring 101 are likewise mounted like the coupling part 10 and the convex ring 1 shown in figs. 1-2.

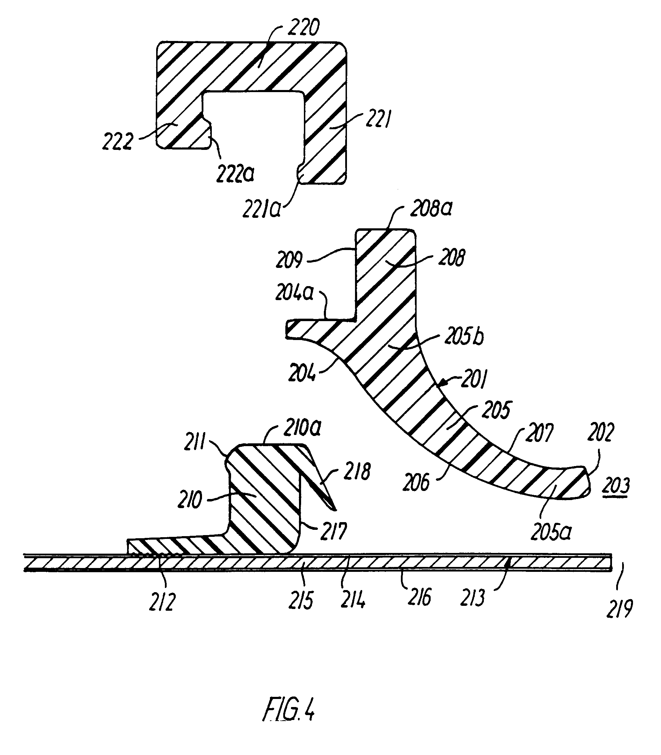

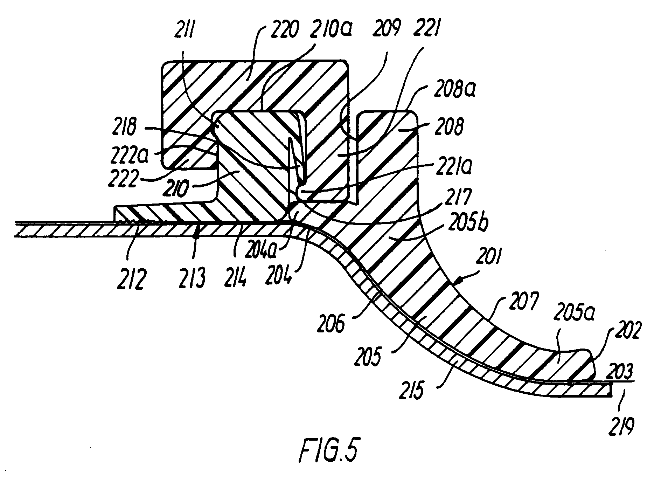

Figs. 4 and 5 show a sketch of a third embodiment of the convex ring of the invention together with or mounted in an ostomy appliance. The ostomy appliance consists of two coupling parts. A first coupling part 210 having an upper distal face 210a and an inner side 217 having an annular flange or lip 218 and a protruding annular flange 211 on its outer side. The coupling part is secured by welding or glueing 212 to a film 214 of an adhesive plate 213 consisting of the film layer 214 and an adhesive layer 215 and having an inner opening 219, said adhesive layer being coated with a removable cover layer 216 prior to the mounting of the coupling part 210, as well as a second coupling part 220 carrying in a generally known manner a collection bag (not shown) and being provided with protruding coupling means 221a, 222a arranged on the outer side and the inner side, respectively, of annular parts 221, 222 protruding in a proximal direction. The ostomy appliance and the coupling between the individual parts are described more fully in EP patent application 463359.

The convex ring 201 has an inner periphery 202 surrounding an opening 203 having a circumference which essentially corresponds to or is slightly greater than the circumference of a stoma for which it is to be used. The convex ring 201 moreover has an outer periphery 204 with a flange 204a as well as a proximal side 206 and a distal side 207.

In the section 205, consisting of inner and outer sections 205a, 205b, between the inner and outer peripheries 202, 204, the convex ring 201 has a convex surface on its proximal side 206. On the outer section 205b the convex ring 201 has a part 208 protruding in a distal direction having an upper face 208a and an outer side 209 which is essentially plane.

In the use of the ostomy appliance and the convex ring 201 the ostomist first removes the removable cover paper 216 from the adhesive layer 215 on the first coupling part 210, and then the adhesive plate 213 is adhered to the skin surface that surrounds the crater or the cavity around the stoma. The convex ring 201 is then pressed down into the coupling part 210 by pressure on the upper face 208 of the protruding part until the upper distal face 208a is flush with the upper face 210a of the coupling part. In practice the distance between the inner side 217 of the coupling part and the outer side 209 of the protruding part will just amount to a few millimeters, and if the ostomist thus presses the upper face 208a of the protruding part with his thumbs, the fingers will automatically be stopped when they touch the upper face 210a of the coupling part, and then the two faces 208a, 210a will be flush with each other and the convex ring is positioned correctly. The inner section of the adhesive plate, i.e. the section closest to the inner opening 209, has now obtained a convex shape, and the adhesive layer 215 is in adhesive contact with the skin surface in the cavity or the crater that surrounds the stoma. The second coupling part 220 can then be mounted as shown in fig. 5, the proximally protruding part 221 of the second coupling part being pressed down into the gap between the protruding part 208 of the convex ring and the inner side 217 of the first coupling part, so that the protruding coupling means 221a engages the lip 218 and the second coupling means 222a engages a protruding flange 211. The coupling parts 210, 220 should have parts capable of engaging each other and providing a "safe" coupling, since the convex ring exerts a pressure in a distal direction toward the second coupling part 220, and since the coupling part is likely to fall off if it is not secured sufficiently. The previously mentioned EP patent application 463359 describes an ostomy appliance which essentially has coupling parts which, when coupled together, are sufficiently secured to each other to withstand the pressure from a convex ring like the one shown in figs. 4 and 5.

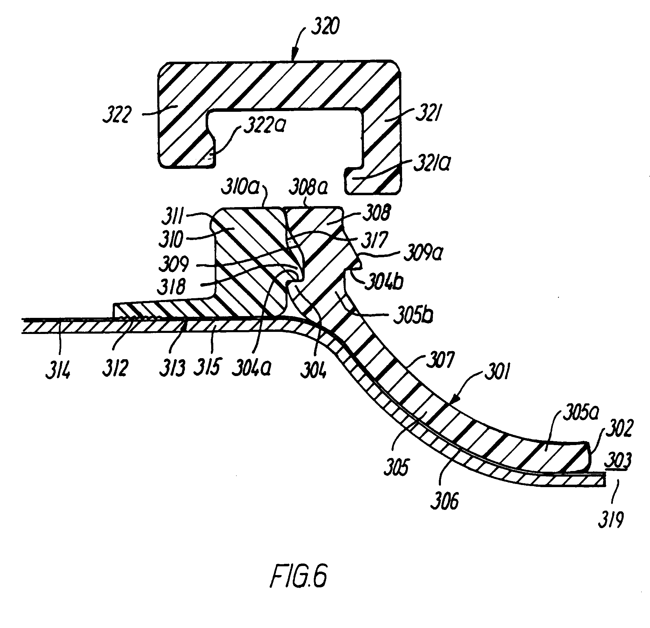

Fig. 6 shows a sketch of a fourth embodiment of the convex ring of the invention mounted in the first coupling part 310 of an ostomy appliance, as well as the second coupling part 320 of the ostomy appliance which carries a collection bag (not shown). The first coupling part 310 has an upper distal face 310a and an inner side 317 having an annular flange or edge 318 and an annular flange or edge 311 on the outer side thereof. The coupling part 310 is secured by welding or glueing 312 to a film 314 of an adhesive plate 313 consisting of the film layer 314 and an adhesive layer 315 and having an inner opening 319. The second coupling part 320 is provided with protruding coupling means 321a, 322a arranged on the outer side and the inner side, respectively, of annular parts 321, 322 protruding in a proximal direction.

The convex ring 301 has an inner periphery 302 that surrounds an opening 303 having a circumference which essentially corresponds to or is slightly greater than the circumference of a stoma for which it is to be used. The convex ring 301 moreover has an outer periphery 304 with a flange or an edge 304a, as well as a proximal side 306 and a distal side 307.

In the section 305, consisting of inner and outer sections 305a, 305b, between the inner and outer peripheries 302, 304, the convex ring 301 has a convex surface on its proximal side 306. On the outer section 305b the convex ring 301 has a distally protruding part 308 having an upper face 308a, an outer side 309 and an inner side 309a having a radially inwardly protruding edge or flange 304b.

When the convex ring 301 is mounted in the first coupling part 310, as shown in fig. 6, the flange or edge 304a of the convex ring engages the edge or flange 318 of the first coupling part, and the protruding part 308 of the convex ring protrudes at a height such that the upper surface 308a of the protruding part is flush with the distal surface 310a of the first coupling part. In the mounting the ostomist has thus pressed on the upper surface 308a of the protruding part with his fingers until the fingers were stopped by contact with the distal surface 310a of the first coupling part, whereby the convex ring was mounted correctly. The second coupling part 320 can then be mounted so that the protruding coupling means 321a engages the radially inwardly protruding edge or flange 304b of the inner side of the convex ring and the coupling means 322a of the second coupling part engages the flange or edge 311 of the first coupling part.

The ostomy appliance shown in fig. 6 can only be used with a convex ring 310 having a distally protruding part 308 of the shown type, the protruding part constituting a coupling means between the first and the second coupling parts 310, 320. If the ostomy appliance with the two coupling parts 310, 320 is to be used by ostomists having a stoma which is not recessed, the convex ring may e.g. be replaced by a ring which exclusively consists of the outer section 305b and the protruding part 308, or the ostomist having the non-recessed stoma can use the first coupling part 310 together with a second coupling part which has a different size and is specially designed for use without a convex ring or the second coupling part 320 together with a first coupling part of a different size specially designed for use without a convex ring.

Thus, the two last-mentioned ostomy appliances will in principle correspond to the ostomy appliance which is shown in EP patent application 463359, fig. 8.

## Claims

1. A convex ring in combination with an ostomy appliance consisting of at least two parts, a first coupling part (10, 110, 210, 310) having a proximal side being intended for adhesive attachment to the skin around the patient's stoma and comprising a ring-shaped part protruding in a distal direction, said ring (1, 101, 201, 301) being of a relatively rigid material and having a wall section (5, 205, 305) extending between an inner periphery (2, 102, 202, 302) and an outer periphery (4, 104, 204, 304), said wall section consisting of an inner wall section (5a, 105a, 205a, 305a) and an outer wall section (5b, 105b, 205b, 305b), a proximal side (6, 106, 206, 306) and a distal side (7, 107, 207, 307), at least the proximal side of said inner wall section having a convex surface, **characterized** in that the two parts of the ostomy appliance are adapted to be coupled together, in that said outer wall section (5b, 105b, 205b, 305b) has at its outer periphery an edge or a flange (4a, 104a, 204a, 304a) capable of engaging an edge or a flange or a plurality of knobs (18, 118, 218, 318) on a radially inner side (17, 117, 217, 317) of the ring-shaped part of the first coupling part when the ring is pressed in proximal direction into said first coupling part, and in that on the outer wall section (5b, 105b, 205b, 305b) the ring (1, 101, 201, 301) has a part (8, 108, 208, 308) protruding in a distal direction.

2. A ring according to claim 1, **characterized** in that the protruding part (208, 308) on the outer wall section (205b, 305b) protrudes at a height such that the upper surface (208a, 308a) is flush with the distal side (210a, 310a) of the ring-shaped part of the first coupling part when the ring (201, 301) and the first coupling part (210, 310) are in mutual engagement.

3. A ring according to claim 1 or 2 in combination with an ostomy appliance consisting of two parts adapted to be coupled together, wherein the second coupling part (220) comprises a radially inner ring-shaped part (221) and a radially outer ring-shaped part, said two ring-shaped parts protruding in a proximal direction, **characterized** in that there is a gap between the inner side of the ring-shaped part (217) of the first coupling part and the outer side of the protruding part (209) of the outer wall section when the ring (201) is in engagement with the first coupling part (210), and that in size the gap corresponds to the thickness of the inner ring-shaped part (221) on the second coupling part (220).

4. A ring according to claim 1, **characterized** in that the protruding part (8, 108) of the outer wall section protrudes over the first coupling part (10, 110) and extends at least a distance inwardly over the distal side (10a, 110a) of the first coupling part when the ring is in engagement with the first coupling part.

5. A ring according to claim 1, 2 or 4, **characterized** in that the first coupling part (110, 310) is caused to engage a second coupling part (120, 320) by means of a third coupling part (130, 308).

6. A ring according to claim 5, **characterized** in that the third coupling part (130) is a locking ring whose radially inner side can be caused to engage the outer side of the ring-shaped part of the first coupling part and an outer side of a proximally protruding ring-shaped part on the second coupling part.

7. A ring according to claim 5, **characterized** in that the third coupling part (308) is formed by the distally protruding part of the convex ring, whose inner side (309) and outer side (309a), respectively, can be caused to engage the first and the second coupling part (310, 320), respectively.

## Patentansprüche

1. Konvexer Ring in Kombination mit einer Stomaeinrichtung, die aus zumindest zwei Teilen besteht, wobei das erste Kupplungsteil (10, 110, 210, 310) eine körpernahe Seite hat, die für eine Klebanbringung an der Haut um das Stoma des Patienten bestimmt ist und ein ringförmiges Teil aufweist, das in eine entfernte Richtung vorsteht, wobei der Ring (1, 101, 201, 301) aus einem verhältnismäßig steifen Material ist und einen Wandabschnitt (5, 205, 305) hat, der sich zwischen einem inneren Rand (2, 102, 202, 302) und einem äußeren Rand (4, 104, 204, 304) erstreckt, wobei der Wandabschnitt aus einem inneren Wandabschnitt (5a, 105a, 205a, 305a) und einem äußeren Wandabschnitt (5b, 105b, 205b, 305b), einer körpernahen Seite (6, 106, 206, 306) und einer entfernten Seite (7, 107, 207, 307) besteht, wobei zumindest die körpernahe Seite des inneren Wandabschnitts eine konvexe Oberfläche hat,
**dadurch gekennzeichnet, daß**
die beiden Teile der Stomaeinrichtung so angepaßt sind, daß sie zusammengekuppelt werden, indem der äußere Wandabschnitt (5b, 105b, 205b, 305b) an seinem äußeren Rand eine Kante oder einen Flansch (4a, 104a, 204a, 304a) hat, der mit einer Kante oder einem Flansch oder einer Vielzahl Nasen (18, 118, 218, 318) an einer radial inneren Seite (17, 117, 217, 317) des ringförmigen Teils des ersten Kupplungsteils in Eingriff gelangen kann, wenn der Ring in eine körpernahe Richtung in das erste Kupplungsteil gedrückt wird, und daß der Ring (1, 101, 201, 301) an dem äußeren Wandabschnitt (5b, 105b, 205b, 305b) ein Teil (8, 108, 208, 308) hat, das in eine entfernte Richtung vorsteht.

2. Ring nach Anspruch 1,
**dadurch gekennzeichnet, daß**
das vorstehende Teil (208, 308) an dem äußeren Wandabschnitt (205b, 305b) in einer derartigen Höhe vorsteht, daß die obere Fläche (208a, 308a) mit der entfernten Seite (210a, 310a) des ringförmigen Teils des ersten Kupplungsteils fluchtet, wenn der Ring (201, 301) und das erste Kupplungsteil (210, 310) in einem gegenseitigen Eingriff sind.

3. Ring nach Anspruch 1 oder 2 in Kombination mit einer Stomaeinrichtung, die aus zumindest zwei Teilen besteht, die so angepaßt sind, daß sie zusammengekuppelt werden, wobei das zweite Kupplungsteil (220) ein radial inneres ringförmiges Teil (221) und ein radiales äußeres ringförmiges Teil aufweist, wobei die beiden ringförmigen Teile in eine körpernahe Richtung vorstehen,
**dadurch gekennzeichnet, daß**
es einen Spalt zwischen der inneren Seite des ringförmigen Teils (217) des ersten Kupplungsteils und der äußeren Seite des vorstehenden Teils (209) des äußeren Wandabschnitts gibt, wenn der Ring (201) mit dem ersten Kupplungsteil (210) im Eingriff ist, und daß die Größe des Spaltes der Dicke des inneren ringförmigen Teils (221) an dem zweiten Kupplungsteil (220) entspricht.

4. Ring nach Anspruch 1,
**dadurch gekennzeichnet, daß**
das vorstehende Teil (8, 108) des äußeren Wandabschnitts über das erste Kupplungsteil (10, 110) vorsteht und sich zumindest in einem Abstand nach innen über die entfernte Seite (10a, 110a) des ersten Kupplungsteils erstreckt, wenn der Ring mit dem ersten Kupplungsteil im Eingriff ist.

5. Ring nach Anspruch 1, 2 oder 4,
**dadurch gekennzeichnet, daß**
bewirkt wird, daß das erste Kupplungsteil (110, 310) mit dem zweiten Kupplungsteil (120, 320) mittels eines dritten Kupplungsteils (130, 308) in Eingriff gelangt.

6. Ring nach Anspruch 5,
**dadurch gekennzeichnet, daß**
das dritte Kupplungsteil (130) ein Verschlußring ist, dessen radial innere Seite bewirken kann, daß die äußere Seite des ringförmigen Teils des ersten Kupplungsteils und eine äußere Seite eines körpernah vorstehenden ringförmigen Teiles an dem zweiten Kupplungsteil in einen Eingriff gelangen.

7. Ring nach Anspruch 5,
**dadurch gekennzeichnet, daß**
das dritte Kupplungsteil (308) durch das entfernt vorstehende Teil des konvexen Ringes gebildet ist, dessen jeweils innere Seite (309) und äußere Seite (309a) bewirken können, daß jeweils das erste und das zweite Kupplungsteil (310, 320) in einen Eingriff gelangen.

## Revendications

1. Anneau convexe associé à un appareil d'ostomie constitué d'au moins deux parties, une première partie de couplage (10, 110, 210, 310) ayant un côté proximal destiné à être attaché de manière adhésive à la peau autour de la stomie du patient et comprenant une partie en forme d'anneau faisant saillie dans une direction distale, ledit anneau (1, 101, 201, 301) étant en matériau relativement rigide et ayant une section de paroi (5, 205, 305) s'étendant entre une périphérie interne (2, 102, 202, 302) et une périphérie externe (4, 104, 204, 304), ladite section de paroi étant constituée d'une section de paroi interne (5a, 105a, 205a, 305a) et d'une section de paroi externe (5b, 105b, 205b, 305b), d'un côté proximal (6, 106, 206, 306) et d'un côté distal (7, 107, 207, 307), au moins le côté proximal de ladite section de paroi interne ayant une surface convexe, caractérisé en ce que les deux parties de l'appareil d'ostomie sont adaptées pour être couplées ensemble, en ce que ladite section de paroi externe (5b, 105b, 205b, 305b) a, au niveau de sa périphérie externe, un bord ou une bride (4a, 104a, 204a, 304a) capable d'engager un bord ou une bride ou une pluralité de projections (18, 118, 218, 318) sur un côté radialement interne (17, 117, 217, 317) de la partie en forme d'anneau de la première partie de couplage lorsque l'anneau est pressé dans une direction proximale dans ladite première partie de couplage et en ce que sur la section de paroi externe (5b, 105b, 205b, 305b), l'anneau (1, 101, 201, 301) a une partie (8, 108, 208, 308) faisant saillie dans une direction distale.

2. Anneau selon la revendication 1, caractérisé en ce que la partie en saillie (208, 308) sur la section de paroi externe (205b, 305b) fait saillie à une hauteur telle que la surface supérieure (208a, 308a) est alignée avec le côté distal (210a, 310a) de la partie en forme d'anneau de la première partie de couplage lorsque l'anneau (201, 301) et la première partie de couplage (210, 310) sont en engagement mutuel.

3. Anneau selon la revendication 1 ou 2, associé à un appareil d'ostomie constitué de deux parties adaptées pour être couplées l'une à l'autre, dans lequel la deuxième partie de couplage (220) comprend une partie en forme d'anneau radialement interne (221) et une partie en forme d'anneau radialement externe, lesdites deux parties en forme d'anneau faisant saillie dans une direction proximale, caractérisé en ce qu'il y a un espace entre le côté interne de la partie en forme d'anneau (217) de la première partie de couplage et le côté externe de la partie en saillie (209) de la section de paroi externe lorsque l'anneau (201) est en engagement avec la première partie de couplage (210), et en ce que, pour ce qui est de la dimension, l'espace correspond à l'épaisseur de la partie en forme d'anneau interne (221) sur la deuxième partie de couplage (220).

4. Anneau selon la revendication 1, caractérisé en ce que la partie en saillie (8, 108) de la section de paroi externe fait saillie par-dessus la première partie de couplage (10, 110) et s'étend au moins sur une certaine distance vers l'intérieur par-dessus le côté distal (10a, 110a) de la première partie de couplage lorsque l'anneau est en engagement avec la première partie de couplage.

5. Anneau selon la revendication 1, 2 ou 4, caractérisé en ce que la première partie de couplage (110, 310) est amenée à engager une deuxième partie de couplage (120, 320) au moyen d'une troisième partie de couplage (130, 308).

6. Anneau selon la revendication 5, caractérisé en ce que la troisième partie de couplage (130) est un anneau de verrouillage dont le côté radialement interne peut être amené à engager le côté externe de la partie en forme d'anneau de la première partie de couplage et un côté externe d'une partie en forme d'anneau faisant saillie proximalement sur la deuxième partie de couplage.

7. Anneau selon la revendication 5, caractérisé en ce que la troisième partie de couplage (308) est formée par la partie de l'anneau convexe faisant saillie distalement, dont le côté interne (309) et le côté externe (309a) respectivement, peuvent être amenés à engager respectivement la première et la deuxième parties de couplage (310, 320).
